(19) Europäisches Patentamt
European Patent Office
Office européen des brevets

(11) EP 4 397 693 A1

(12) **EUROPEAN PATENT APPLICATION**
published in accordance with Art. 153(4) EPC

(43) Date of publication:
**10.07.2024 Bulletin 2024/28**

(21) Application number: **22864184.1**

(22) Date of filing: **05.08.2022**

(51) International Patent Classification (IPC):
***C08G 75/08*** (2006.01) ***C07C 319/24*** (2006.01)
***C07C 323/24*** (2006.01) ***C07D 331/02*** (2006.01)
***G02B 1/04*** (2006.01)

(52) Cooperative Patent Classification (CPC):
**C07C 319/24; C07C 323/24; C07D 331/02; C08G 75/08; G02B 1/04**

(86) International application number:
**PCT/JP2022/030174**

(87) International publication number:
**WO 2023/032598 (09.03.2023 Gazette 2023/10)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **01.09.2021 JP 2021142430**

(71) Applicant: **Mitsui Chemicals, Inc.**
**Tokyo 104-0028 (JP)**

(72) Inventors:
- **TOKUNAGA, Koichi**
  **Omuta-shi, Fukuoka 836-8610 (JP)**
- **FURUYA, Masayuki**
  **Omuta-shi, Fukuoka 836-8610 (JP)**

(74) Representative: **Mewburn Ellis LLP**
**Aurora Building**
**Counterslip**
**Bristol BS1 6BX (GB)**

(54) **EPISULFIDE COMPOSITION, POLYMERIZABLE COMPOSITION, CURED PRODUCT, AND OPTICAL MATERIAL**

(57) An episulfide composition, including: an episulfide compound (A) that comprises an episulfide ring, and a polysulfide bond in which three or more sulfide bonds represented by - S- are linked together; and an episulfide compound (B) that comprises an episulfide ring and a (di)sulfide bond but not the polysulfide bond, wherein the content of the episulfide compound (A) is 100 ppm by mass or more with respect to a total amount of the episulfide composition.

EP 4 397 693 A1

## Description

### Technical Field

The present disclosure relates to an episulfide composition, a polymerizable composition, a cured product, and an optical material.

### Background Art

Plastic lenses are lightweight, hardly breakable, and dyeable as compared to inorganic lenses; therefore, in recent years, the use of plastic lenses as eyeglass lenses, camera lenses, and the like has been rapidly expanded.
Among resin-made optical materials represented by plastic lenses, optical materials obtained by polymerization of episulfide compounds are known to have a high refractive index (see, for example, Patent Document 1).
Patent Document 1: Japanese Patent Application Laid-Open (JP-A) No. 2002-194083

## SUMMARY OF THE INVENTION

### Technical Problem

However, it may be required to further improve the polymerizability of an episulfide compound as a monomer (e.g., the episulfide compound having a disulfide bond that is disclosed in Patent Document 1).
An object of the disclosure is to provide: an episulfide composition having excellent polymerizability; a polymerizable composition that contains the episulfide composition and has excellent polymerizability; a cured product of the polymerizable composition; and an optical material including the cured product.

### Solution to Problem

Means for solving the above-described problems encompass the following aspects.

<1> An episulfide composition, comprising:

an episulfide compound (A) that comprises an episulfide ring, and a polysulfide bond in which three or more bonds represented by -S- are linked together; and
an episulfide compound (B) that comprises an episulfide ring and a (di)sulfide bond but not the polysulfide bond, wherein a content of the episulfide compound (A) is 100 ppm by mass or more with respect to a total amount of the episulfide composition.

<2> The episulfide composition according to <1>, wherein the episulfide compound (A) comprises a trisulfide bond.
<3> The episulfide composition according to <1> or <2>, wherein the content of the episulfide compound (A) is 15,000 ppm by mass or less with respect to the total amount of the episulfide composition.
<4> The episulfide composition according to any one of <1> to <3>, wherein the content of the episulfide compound (B) is 90% by mass or more with respect to the total amount of the episulfide composition.
<5> The episulfide composition according to any one of <1> to <4>, wherein the episulfide compound (A) comprises a compound represented by the following Formula (1):

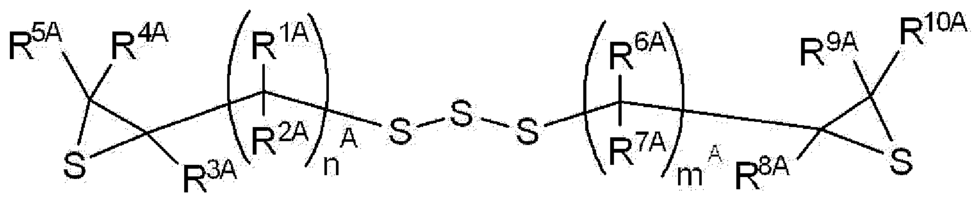

(1)

wherein, in Formula (1), each of $R^{1A}$ to $R^{10A}$ independently represents a hydrogen atom or an optionally substituted monovalent hydrocarbon group, and each of $n^A$ and $m^A$ independently represents an integer from 0 to 3; when $n^A$ is 2 or 3, plural $R^{1A}$s are optionally the same or different, and plural $R^{2A}$s are optionally the same or different; and
when $m^A$ is 2 or 3, plural $R^{6A}$s are optionally the same or different, and plural $R^{7A}$s are optionally the same or

different.

<6> The episulfide composition according to any one of <1> to <5>, wherein the episulfide compound (B) comprises a compound represented by the following Formula (2):

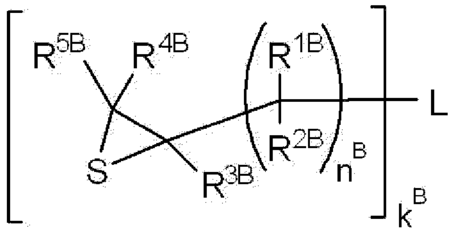

(2)

wherein, in Formula (2), each of $R^{1B}$ to $R^{5B}$ independently represents a hydrogen atom or an optionally substituted monovalent hydrocarbon group, $n^B$ represents an integer from 0 to 3, and $k^B$ represents an integer of 2 to 4;
when $k^B$ is 2, L represents a $k^B$-valent linking group that comprises at least one of a sulfide bond or a disulfide bond and a hydrocarbon group, a sulfide bond, or a disulfide bond;
when $k^B$ is 3 or 4, L represents a $k^B$-valent linking group that comprises at least one of a sulfide bond or a disulfide bond and a hydrocarbon group; and
plural $R^{1B}$s to $R^{5B}$s and $n^B$s are each optionally the same or different.

<7> A polymerizable composition, comprising: the episulfide composition according to any one of <1> to <6>; and a polymerization catalyst.
<8> The polymerizable composition according to <7>, further comprising a thiol compound.
<9> A cured product of the polymerizable composition according to <7> or <8>.
<10> An optical material, comprising the cured product according to <9>.

Advantageous Effects of Invention

According to the disclosure, the following are provided: an episulfide composition having excellent polymerizability; a polymerizable composition that contains the episulfide composition and has excellent polymerizability; a cured product of the polymerizable composition; and an optical material including the cured product.

DESCRIPTION OF EMBODIMENTS

In the disclosure, those numerical ranges that are expressed with "to" each denote a range that includes the numerical values stated before and after "to" as the lower limit value and the upper limit value, respectively.
In the disclosure, when there are plural substances that correspond to a component of a composition, the indicated amount of the component in the composition means, unless otherwise specified, a total amount of the plural substances existing in the composition.
In a set of numerical ranges that are stated in a stepwise manner in the disclosure, the upper limit value or the lower limit value of one numerical range may be replaced with the upper limit value or the lower limit value of other numerical range. Further, in a numerical range stated in the disclosure, the upper limit value or the lower limit value of the numerical range may be replaced with a relevant value indicated in any of Examples.

[Episulfide Composition]

The episulfide composition of the disclosure is an episulfide composition containing:

an episulfide compound (A) that contains an episulfide ring, and a polysulfide bond in which three or more bonds represented by -S- are linked together; and
an episulfide compound (B) that contains an episulfide ring and a (di)sulfide bond but not a trisulfide bond,
wherein the content of the episulfide compound (A) is 100 ppm by mass or more with respect to a total amount of the episulfide composition.

The episulfide composition of the disclosure has excellent polymerizability.
It is believed that the episulfide compound (A), which is contained in an amount of 100 ppm by mass or more

with respect to a total amount of the episulfide composition, contributes to the excellent polymerizability.

The term "(di)sulfide bond" used herein is a concept that encompasses a sulfide bond and a disulfide bond.

The term "sulfide bond" used herein means a bond represented by "-S-" linking two carbon atoms (excluding a bond represented by "-S-" contained in an episulfide ring).

The term "disulfide bond" used herein means a bond represented by "-S-S-" linking two carbon atoms.

In the disclosure, the term "polysulfide bond in which three or more bonds represented by -S- are linked together" (hereinafter, also simply referred to as "polysulfide bond") means a bond represented by "$-(S)_n-$" (wherein, n represents an integer of 3 or larger) that links two carbon atoms.

In the disclosure, examples of a polysulfide bond include a trisulfide bond, a tetrasulfide bond, a pentasulfide bond, and a hexasulfide bond.

Here,

"trisulfide bond" means a bond represented by "-S-S-S-" that links two carbon atoms;
"tetrasulfide bond" means a bond represented by "-S-S-S-S-" that links two carbon atoms;
"pentasulfide bond" means a bond represented by "-S-S-S-S-S-" that links two carbon atoms; and
"hexasulfide bond" means a bond represented by "-S-S-S-S-S-S-" that links two carbon atoms.

Components that may be contained in the episulfide composition of the disclosure will now be described.

<Episulfide Compound (A)>

The episulfide composition of the disclosure contains an episulfide compound (A).

The episulfide compound (A) is a compound that contains an episulfide ring, and a polysulfide bond in which three or more bonds represented by -S- are linked together.

The episulfide composition of the disclosure may contain the episulfide compound (A) singly, or in combination of two or more kinds thereof.

The content of the episulfide compound (A) is 100 ppm by mass or more with respect to a total amount of the episulfide composition of the disclosure.

By this, the polymerizability of the episulfide composition is improved.

It is noted here that the concept of the term "polymerizability of the episulfide composition" encompasses not only the polymerizability in polymerization of only the episulfide compounds (A) and (B) contained in the episulfide composition, but also the polymerizability in copolymerization of the episulfide compounds (A) and (B) with other monomer (e.g., the below-described thiol compound; the same applies hereinafter).

From the standpoint of further improving the polymerizability of the episulfide composition, the content of the episulfide compound (A) is preferably 200 ppm by mass or more, more preferably 300 ppm by mass or more, still more preferably 500 ppm by mass or more, yet still more preferably 1,000 ppm by mass or more.

From the standpoint of maintaining the resin properties of the resulting resin in a more favorable manner, the content of the episulfide compound (A) is preferably 15,000 ppm by mass or less with respect to a total amount of the episulfide composition.

It is noted here that the concept of the term "resulting resin" encompasses not only a resin obtained by polymerizing only the episulfide compounds (A) and (B) contained in the episulfide composition, but also a resin obtained by copolymerizing the episulfide compounds (A) and (B) with other monomer (e.g., the below-described thiol compound; the same applies hereinafter).

Examples of the resin properties include light resistance and heat resistance.

In the below-described Examples, the heat resistance was evaluated using the following as indices:

glass transition temperature (Tg); and
$\Delta$Y.I., which is a difference in yellow index (Y.I.) before and after annealing.

From the standpoint of further improving the resin properties of the resulting resin, the content of the episulfide compound (A) is preferably 12,000 ppm by mass or less, more preferably 10,000 ppm by mass or less, still more preferably 8,000 ppm by mass or less, yet still more preferably 5,000 ppm by mass or less.

The episulfide compound (A) preferably contains a trisulfide bond.

In other words, the episulfide compound (A) preferably contains an episulfide compound (Aa) containing an episulfide ring and a trisulfide bond.

In this case, the episulfide compound (A) may contain:

the episulfide compound (Aa) containing an episulfide ring and a trisulfide bond; and

an episulfide compound (Ab) containing an episulfide ring and a polysulfide bond other than a trisulfide bond (e.g., a tetrasulfide bond).

The episulfide compound (Aa) may also contain a trisulfide bond and a polysulfide bond other than the trisulfide bond (e.g., a tetrasulfide bond).
When the episulfide compound (A) contains the episulfide compound (Aa), a preferred range of the content of the episulfide compound (Aa) is the same as the above-described preferred range of the content of the episulfide compound (A).
Further, when the episulfide compound (A) contains the episulfide compound (Aa), a ratio of the episulfide compound (Aa) in a total amount of the episulfide compound (A) is preferably from 50% by mass to 100% by mass, more preferably from 60% by mass to 100% by mass, still more preferably from 80% by mass to 100% by mass.
The episulfide compound (A) more preferably contains a compound represented by the following Formula (1).
When the episulfide compound (A) contains the compound represented by the following Formula (1), a preferred range of the content of the compound represented by the following Formula (1) is the same as the above-described preferred range of the content of the episulfide compound (A).
Further, when the episulfide compound (A) contains the compound represented by the following Formula (1), a ratio of the compound represented by the following Formula (1) in a total amount of the episulfide compound (A) is preferably from 50% by mass to 100% by mass, more preferably from 60% by mass to 100% by mass, still more preferably from 80% by mass to 100% by mass, yet still more preferably from 90% by mass to 100% by mass, further preferably from 95% by mass to 100% by mass.

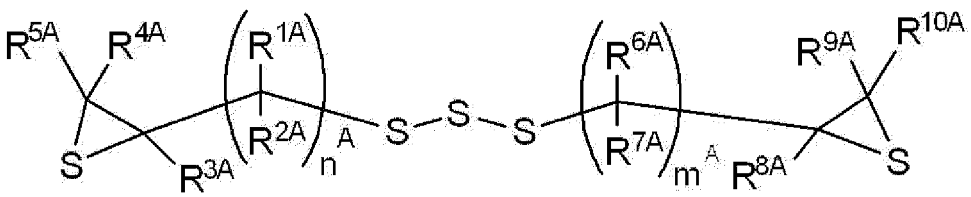

(1)

In Formula (1), each of $R^{1A}$ to $R^{10A}$ independently represents a hydrogen atom or an optionally substituted monovalent hydrocarbon group, and each of $n^A$ and $m^A$ independently represents an integer from 0 to 3.
When $n^A$ is 2 or 3, plural $R^{1A}$s are optionally the same or different, and plural $R^{2A}$s are optionally the same or different.
When $m^A$ is 2 or 3, plural $R^{6A}$s are optionally the same or different, and plural $R^{7A}$s are optionally the same or different.
In Formula (1), each of $R^{1A}$ to $R^{10A}$ is independently a hydrogen atom or an optionally substituted monovalent hydrocarbon group.
Examples of a substituent in the optionally substituted monovalent hydrocarbon group include a halogen atom, a thiol group (i.e., a mercapto group), an alkoxy group, an alkylthio group, and a hydroxy group.
When a substituent is contained, the number of carbon atoms of the optionally substituted monovalent hydrocarbon group is preferably from 1 to 10, more preferably from 1 to 6, still more preferably from 1 to 3, yet still more preferably 1 or 2, further preferably 1, in terms of a total number of carbon atoms that includes the number of carbon atoms of the substituent.
The "optionally substituted monovalent hydrocarbon group" represented by each of $R^{1A}$ to $R^{10A}$ in Formula (1) is preferably an alkyl group having from 1 to 6 carbon atoms (i.e., an unsubstituted alkyl group), more preferably an alkyl group having from 1 to 3 carbon atoms, still more preferably a methyl group or an ethyl group, yet still more preferably a methyl group.
In Formula (1), $R^{1A}$ to $R^{10A}$ are each particularly preferably a hydrogen atom.
In Formula (1), each of $n^A$ and $m^A$ is independently an integer from 0 to 3.
Each of $n^A$ and $m^A$ is independently preferably an integer from 0 to 2, more preferably 0 or 1, still more preferably 1.
An example of a method of producing the episulfide compound (A) of an aspect containing a trisulfide bond (e.g., a compound represented by Formula (1)) will now be described (this method is hereinafter referred to as "production method A").
The production method A includes:

the step (a1) of reacting two molecules (which are optionally the same or different) of the below-described compound (A-1) containing a halogen atom and a mercapto group (e.g., 1-chloro-3-mercapto-2-propanol) in the presence of

sulfur, a basic compound (A-2) (e.g., NaSH), and/or a basic compound (A-3) at a charged molar ratio of sulfur of higher than 1.00 with respect to the compound (A-1), to generate the below-described compound (A-4) containing a halogen atom and a trisulfide bond;
the step (a2) of epoxidizing the compound (A-4) under a basic condition to generate the below-described compound (A-5) containing an epoxy group and a trisulfide bond; and
the step (a3) of reacting the compound (A-5) with a sulfurizing agent to generate the above-described compound represented by Formula (1) as an episulfide compound (A) (i.e., a compound containing an episulfide ring and a trisulfide bond).

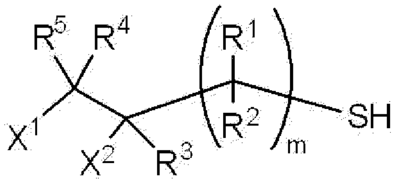
(A-1)

$M(SH)_n$ (A-2)

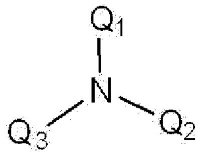
(A-3)

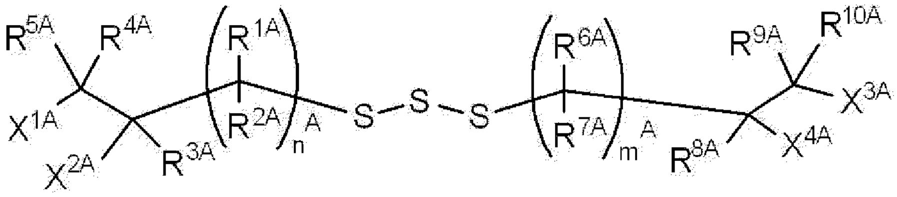
(A-4)

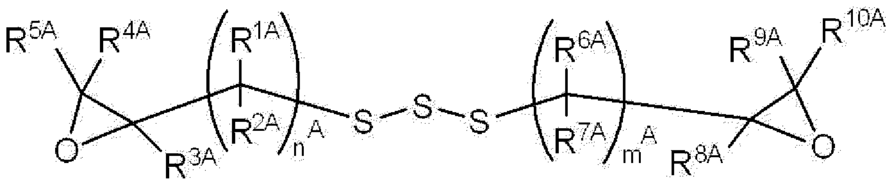
(A-5)

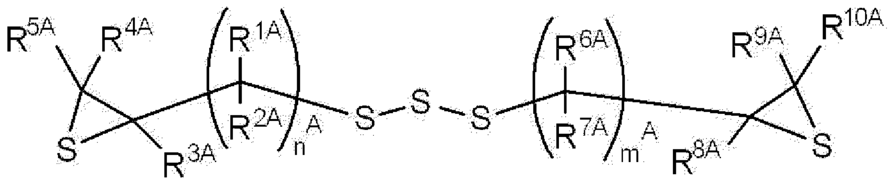
(1)

In the compound (A-1), each of $R^1$ to $R^5$ independently represents a hydrogen atom or an optionally substituted monovalent hydrocarbon group, m represents an integer from 0 to 3, and one of $X^1$ and $X^2$ is a hydroxy group while the other is a halogen atom.

When m is 2 or 3, plural $R^1$s are optionally the same or different, and plural $R^2$s are optionally the same or different.

In the compound (A-1), preferred aspects of $R^1$ to $R^5$ and m are the same as the preferred aspects of $R^{1A}$ to $R^{10A}$ and $m^A$ in Formula (1).

In the compound (A-1), the halogen atom represented by $X^1$ or $X^2$ is preferably a fluorine atom, a chlorine atom, a bromine atom, or an iodine atom, particularly preferably a chlorine atom.

In the compound (A-2), M represents an alkali metal or an alkaline earth metal, and n represents a valence of M (i.e., 1 or 2).

In the compound (A-2), M is preferably an alkali metal such as lithium, sodium, or potassium, more preferably sodium.

Examples of the compound (A-2) include sodium hydrogen sulfide (NaSH), potassium hydrogen sulfide, magnesium hydrogen sulfide, and calcium hydrogen sulfide. Thereamong, it is preferred to use sodium hydrogen su [0038] In the compound (A-3), each of Q1, Q2, and Q3 independently represents a hydrogen atom, a linear or branched aliphatic group having from 1 to 20 carbon atoms, an alicyclic group having from 3 to 20 carbon atoms, or a substituted or unsubstituted aromatic group, and these groups optionally contain a heteroatom.

Examples of the linear or branched aliphatic group having from 1 to 20 carbon atoms include: linear or branched alkyl groups, such as a methyl group, an ethyl group, a butyl group, an isobutyl group, an octyl group, and a 2-ethylhexyl group; and linear or branched alkenyl groups, such as a vinyl group (an ethenyl group), a butenyl group, and an octenyl group.lfide (NaSH).

In the compound (A-3), each of $Q^1$, $Q^2$, and $Q^3$ independently represents a hydrogen atom, a linear or branched aliphatic group having from 1 to 20 carbon atoms, an alicyclic group having from 3 to 20 carbon atoms, or a substituted or unsubstituted aromatic group, and these groups optionally contain a heteroatom.

Examples of the linear or branched aliphatic group having from 1 to 20 carbon atoms include: linear or branched alkyl groups, such as a methyl group, an ethyl group, a butyl group, an isobutyl group, an octyl group, and a 2-ethylhexyl group; and linear or branched alkenyl groups, such as a vinyl group (an ethenyl group), a butenyl group, and an octenyl group.

Examples of the alicyclic group having from 3 to 20 carbon atoms include a cyclopropyl group, a cyclopentyl group, a cyclohexyl group, a cyclohexenyl group, a tetrahydropyranyl group, a tetrahydrofuranyl group, a tetrahydrothiopyranyl group, a tetrahydrothiofuranyl group, a 3-bromotetrahydropyranyl group, a 4-methoxytetrahydropyranyl group, a 4-methoxytetrahydrothiopyranyl group, a 3-tetrahydrothiophene-1,1-dioxide group, a 2-methyl-2-adamantyl group, and a 2-ethyl-2-adamantyl group.

Examples of the substituted or unsubstituted aromatic group include a phenyl group, a tolyl group, and a xylyl group.

$Q^1$, $Q^2$, and $Q^3$ are preferably the same with each other, more preferably linear or branched aliphatic groups having from 1 to 20 carbon atoms, particularly preferably ethyl groups.

Examples of the compound (A-3) include ammonia, methylamine, ethylamine, n-propylamine, isopropylamine, n-butylamine, s-butylamine, t-butylamine, dimethylamine, diethylamine, ethylmethylamine, aniline, *N*-methylaniline, *N*-ethylaniline, trimethylamine, triethylamine, tri-*n*-propylamine, triisopropylamine, tri-*n*-butylamine, tri-*n*-cyclohexylamine, *N*,*N*-diisopropylethylamine, *N*,*N*-dimethyl-*n*-octadecylamine, triethylenediamine, triphenylamine, *N*,*N*-dimethylethanolamine, *N*,*N*-diethylethanolamine, triethanolamine, *N*,*N*-dimethylcyclohexylamine, *N*,*N*-diethylcyclohexylamine, *N*,*N*-dimethylbutylamine, *N*-methyldicyclohexylamine, *N*,*N*-dimethylaniline, *N*,*N*-diethylaniline, and tetramethylethylenediamine.

Thereamong, triethylamine is preferred.

In the compound (A-4), $R^{1A}$ to $R^{10A}$, $n^A$, and $m^A$ have the same meaning as $R^{1A}$ to $R^{10A}$, $n^A$, and $m^A$ of Formula (1), respectively, and their preferred aspects are also the same.

In the compound (A-4), one of $X^{1A}$ and $X^{2A}$ is a hydroxy group while the other is a halogen atom.

In the compound (A-4), one of $X^{3A}$ and $X^{4A}$ is a hydroxy group while the other is a halogen atom.

A preferred aspect of the halogen atom represented by each of $X^{1A}$ to $X^{4A}$ is the same as that of the halogen atom represented by each of $X^1$ and $X^2$ in the compound (A-1).

In the compound (A-5), $R^{1A}$ to $R^{10A}$, $n^A$, and $m^A$ have the same meaning as $R^{1A}$ to $R^{10A}$, $n^A$, and $m^A$ of Formula (1), respectively, and their preferred aspects are also the same.

According to the production method A, the episulfide compound (A) of an aspect containing a trisulfide bond (e.g., a compound represented by Formula (1)) can be easily produced by controlling the charged molar ratio of sulfur with respect to the compound (A-1) to be higher than 1.0 (preferably 1.1 or higher).

In the production method A, a mixture of the episulfide compound (A) and the episulfide compound (B) may be obtained as well.

-Preferred Aspect of Step (a1)-

The step (a1) is a step of reacting two molecules (which are optionally the same or different) of the compound (A-1) in the presence of sulfur, the basic compound (A-2), and/or the basic compound (A-3) at a charged molar ratio of sulfur of higher than 1.00 with respect to the compound (A-1), to generate the compound (A-4).

The step (a1) can be performed in a reaction solvent and, as the reaction solvent, for example, aromatic solvents such as toluene, xylene, chlorobenzene, dichlorobenzene, and nitrobenzene, aliphatic solvent such as dichloromethane, chloroform, and dichloroethane, alcohols such as methanol, ethanol, isopropanol, butanol, methoxy ethanol, ethylene glycol, and glycerol, and water can be used. The reaction solvent is particularly preferably water. The above-described reaction solvents may be used singly, or in combination of two or more kinds thereof as a mixture.

The charged molar ratio of sulfur with respect to the compound (A-1) is higher than 1.00, preferably 1.02 or

higher, more preferably 1.03 or higher, still more preferably 1.04 or higher.

The charged molar ratio of sulfur with respect to the compound (A-1) is preferably 1.50 or lower, more preferably 1.20 or lower, still more preferably 1.10 or lower.

A total charged amount of the basic compound (A-2) and/or the basic compound (A-3) is preferably from 0.01% by mole to 1.0% by mole, more preferably from 0.05% by mole to 0.5% by mole, particularly preferably from 0.10% by mole to 0.30% by mole, with respect to the compound (A-1).

The reaction temperature is preferably from 3°C to 20°C, more preferably from 5°C to 15°C.

The reaction time is not particularly limited; however, it is, for example, from 3 hours to 20 hours.

The pH is also not particularly limited; however, it is preferably from 7.5 to 10, more preferably from 8.0 to 9.0.

-Preferred Aspect of Step (a2)-

The step (a2) is a step of epoxidizing the compound (A-4) under a basic condition to generate the compound (A-5) containing an epoxy group and a trisulfide bond.

Examples of a base used in the basic condition include ammonia, triethylamine, tributylamine, dimethylcyclohexylamine, diethylaniline, pyridine, sodium hydroxide, potassium hydroxide, magnesium hydroxide, calcium hydroxide, sodium carbonate, sodium bicarbonate, potassium carbonate, sodium methylate, *t*-butoxy potassium, disodium monohydrogen phosphate, and sodium acetate.

These bases may be used singly, or in combination of two or more kinds thereof.

As the type of the base, inorganic bases are more preferred than organic bases and, among inorganic bases, sodium hydroxide and potassium hydroxide are preferred.

The amount of these bases to be used is, in terms of the charged molar ratio of the bases with respect to the compound (A-1), preferably from 1.00 to 2.00, more preferably from 1.10 to 1.50.

The reaction temperature is preferably from -10°C to 60°C, more preferably from 5°C to 30°C.

The step (a2) can be performed in a reaction solvent, and examples of the reaction solvent include:

aromatic solvents, such as toluene, xylene, chlorobenzene, dichlorobenzene, and nitrobenzene;
aliphatic solvent, such as dichloromethane, chloroform, and dichloroethane;
alcohols, such as methanol, ethanol, isopropanol, butanol, methoxy ethanol, ethylene glycol, and glycerol; and water.

The reaction solvent is particularly preferably toluene or water.

The above-described reaction solvents may be used singly, or in combination of two or more kinds thereof as a mixture.

-Preferred Aspect of Step (a3)-

The step (a3) is a step of reacting the compound (A-5) with a sulfurizing agent to generate a compound represented by Formula (1) as an episulfide compound (A).

Examples of the sulfurizing agent include:

thiourea; and
thiocyanates, such as sodium thiocyanate, potassium thiocyanate, ammonium thiocyanate, calcium thiocyanate, and lead thiocyanate.

When a thiocyanate is used, it is preferably sodium thiocyanate, potassium thiocyanate, or ammonium thiocyanate, more preferably sodium thiocyanate.

The amount of the sulfurizing agent to be used is preferably 1 equivalent or more, more preferably from 1 equivalent to 5 equivalents, still more preferably 1 equivalent to 3 equivalents, with respect to the amount of epoxy group.

The reaction temperature varies depending on the type of the sulfurizing agent; however, it is preferably from 10°C to 30°C when thiourea is used, or preferably from 30°C to 60°C when a thiocyanate is used.

The step (a3) can be performed in a reaction solvent.

Examples of the reaction solvent include:

aromatic solvents, such as toluene, xylene, chlorobenzene, dichlorobenzene, and nitrobenzene;
aliphatic solvent, such as dichloromethane, chloroform, and dichloroethane; and
alcohols, such as methanol, ethanol, isopropanol, butanol, methoxy ethanol, ethylene glycol, and glycerol.

These reaction solvents may be used singly, or in combination of two or more kinds thereof as a mixture.

<Episulfide Compound (B)>

The episulfide composition of the disclosure contains an episulfide compound (B).

The episulfide compound (B) is a compound that contains an episulfide ring and a (di)sulfide bond but not the above-described polysulfide bond (i.e., a polysulfide bond in which three or more bonds represented by -S- are linked together).

The episulfide composition of the disclosure may contain the episulfide compound (B) singly, or in combination of two or more kinds thereof.

The episulfide compound (B) contributes to ensuring the resin properties of the resulting resin.

The content of the episulfide compound (B) is preferably 60% by mass or more, more preferably 80% by mass or more, still more preferably 90% by mass or more, yet still more preferably 95% by mass or more, with respect to a total amount of the episulfide composition.

By this, the resin properties of the resulting resin are further improved.

An upper limit of the content of the episulfide compound (B) varies depending on the content of the episulfide compound (A); however, the upper limit of the episulfide compound (B) is, for example, 99% by mass or 98% by mass.

The episulfide compound (B) preferably contains a compound represented by the following Formula (2).

When the episulfide compound (B) contains the compound represented by the following Formula (2), a preferred range of the content of the compound represented by the following Formula (2) is the same as the above-described preferred range of the content of the episulfide compound (B).

Further, when the episulfide compound (B) contains the compound represented by the following Formula (2), a ratio of the compound represented by the following Formula (2) in a total amount of the episulfide compound (B) is preferably from 50% by mass to 100% by mass, more preferably from 60% by mass to 100% by mass, still more preferably from 80% by mass to 100% by mass.

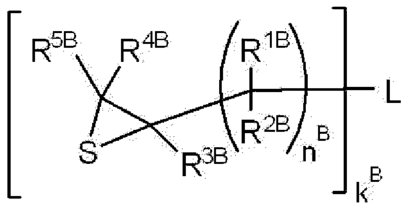

(2)

In Formula (2), each of $R^{1B}$ to $R^{5B}$ independently represents a hydrogen atom or an optionally substituted monovalent hydrocarbon group, $n^B$ represents an integer from 0 to 3, and $k^B$ represents an integer of 2 to 4.

When $k^B$ is 2, L represents a $k^B$-valent linking group that contains at least one of a sulfide bond or a disulfide bond and a hydrocarbon group, a sulfide bond, or a disulfide bond.

When $k^B$ is 3 or 4, L represents a $k^B$-valent linking group that contains at least one of a sulfide bond or a disulfide bond and a hydrocarbon group.

Plural $R^{1B}$s to $R^{5B}$s and $n^B$s are each optionally the same or different.

In Formula (2), each of $R^{1B}$ to $R^{5B}$ is independently a hydrogen atom or an optionally substituted monovalent hydrocarbon group.

A preferred aspect of the "optionally substituted monovalent hydrocarbon group" represented by each of $R^{1B}$ to $R^{5B}$ in Formula (2) is the same as the above-described preferred aspect of the "optionally substituted monovalent hydrocarbon group" represented by each of $R^{1A}$ to $R^{10A}$ in Formula (1).

In Formula (2), $R^{1B}$ to $R^{5B}$ are each particularly preferably a hydrogen atom.

In Formula (2), $n^B$ is an integer from 0 to 3.

In Formula (2), $n^B$ is preferably an integer from 0 to 2, more preferably 0 or 1, still more preferably 1.

In Formula (2), $k^B$ is an integer of 2 to 4.

In Formula (2), $k^B$ is preferably 2.

In Formula (2), when $k^B$ is 2, L is a $k^B$-valent (i.e., divalent) linking group that contains at least one of a sulfide bond or a disulfide bond and a hydrocarbon group, a sulfide bond, or a disulfide bond.

In Formula (2), when $k^B$ is 3 or 4, L is a $k^B$-valent (i.e., trivalent or tetravalent) linking group that contains at least one of a sulfide bond or a disulfide bond and a hydrocarbon group.

In the $k^B$-valent (i.e., trivalent or tetravalent) linking group that contains at least one of a sulfide bond or a disulfide bond and a hydrocarbon group, the hydrocarbon group may be a chain aliphatic group, a cyclic aliphatic group, an aromatic group, or a combination of two or more of these groups.

The $k^B$-valent linking group may contain only one hydrocarbon group, or two or more hydrocarbon groups.
The $k^B$-valent linking group may contain only one of at least one of a sulfide bond or a disulfide bond, or two or more of at least one of a sulfide bond or a disulfide bond.
Further, $k^B$-valent linking group may contain one or more sulfur-containing heterocyclic structures.
The number of carbon (i.e., the number of carbon atoms) in the $k^B$-valent linking group is preferably from 1 to 30, more preferably from 1 to 20, still more preferably from 1 to 10.
The number of sulfur (i.e., the number of sulfur atoms) in the $k^B$-valent linking group is preferably from 1 to 30, more preferably from 1 to 20, still more preferably from 1 to 10.
With regard to specific examples of the $k^B$-valent linking group, reference can be made to, for example, the paragraphs [0034] to [0041] of JP-A No. 2021-91757.
The molecular weight of the compound represented by Formula (2) is preferably 1,000 or less, more preferably 800 or less, still more preferably 500 or less, yet still more preferably 300 or less.
A lower limit of the molecular weight of the compound represented by Formula (2) is, for example, 60, and it is preferably 100, more preferably 130.
A preferred aspect of the compound represented by Formula (2) is an aspect in which $k^B$ is 2 and L is a sulfide bond (-S-) or a disulfide bond (-S-S-) in Formula (2).
One example of a preferred compound among those compounds represented by Formula (2) is a compound in which $k^B$ is 2 and L is a disulfide bond (-S-S-) in Formula (1) (hereinafter, also referred to as "compound (2a)").
Specific examples of this aspect include bis(2,3-epithiopropyl)disulfide, bis(1,2-epithioethyl)disulfide, bis(3,4-epithiobutyl)disulfide, and bis(4,5-epithiopentyl)disulfide, among which bis(2,3-epithiopropyl)disulfide is particularly preferred.
Another example of a preferred compound among those compounds represented by Formula (2) is a compound in which $k^B$ is 2 and L is a sulfide bond (-S-) in Formula (2) (hereinafter, also referred to as "compound (2b)").
Specific examples of this aspect include bis(2,3-epithiopropyl)sulfide, bis(1,2-epithioethyl)sulfide, bis(3,4-epithiobutyl)sulfide, and bis(4,5-epithiopentyl)sulfide, among which bis(2,3-epithiopropyl)sulfide is particularly preferred.
The episulfide compound (B) in the episulfide composition of the disclosure particularly preferably contains at least one of the compound (2A) or the compound (2B).
In this case, a total ratio of the compound (2a) and the compound (2b) in a total amount of the episulfide compound (B) is preferably from 50% by mass to 100% by mass, more preferably from 60% by mass to 100% by mass, still more preferably from 80% by mass to 100% by mass.
Specific examples of the episulfide compound (B) also include the following in addition to the above-described compound (2a) and compound (2b).
Examples of an episulfide compound that contains two or more episulfide rings and a chain aliphatic structure include bis(2,3-epithiopropylthio)methane and 1,2-bis(2,3-epithiopropylthio)ethane.
Examples of an episulfide compound that contains two or more episulfide rings and a cyclic aliphatic structure or a sulfur-containing heterocyclic structure include 1,3-bis(2,3-epithiopropylthio)cyclohexane and 1,4-bis(2,3-epithiopropylthio)cyclohexane.
Examples of an episulfide compound that contains two or more episulfide rings and an aromatic ring structure include 1,2-bis(2,3-epithiopropylthio)benzene and 1,3-bis(2,3-epithiopropylthio)benzene.
Examples of the episulfide compound (B) also include compounds of the above-described specific examples in which at least one sulfide bond (-S-) is replaced with a disulfide bond (-S-S-).
Examples of the episulfide compound (B) further include compounds containing only one episulfide ring, such as ethylene sulfide, propylene sulfide, 3-mercaptopropylene sulfide, and 4-mercaptobutene sulfide.
With regard to the episulfide compound (B) and a production method thereof, reference can be made as appropriate to known documents, such as JP-A No. 2002-194083 and JP-A No. 2021-91757.

<Other Components>

The episulfide composition of the disclosure may contain other components in addition to the episulfide compound (A) and the episulfide compound (B).
Examples of the other components include reaction solvents used for the synthesis of the episulfide compound (A) and the episulfide compound (B), and reaction by-products (impurities) generated by the synthesis.

<Use>

The use of the episulfide composition of the disclosure is not particularly limited.
The episulfide composition of the disclosure can yield a cured product of an episulfide compound that can exhibit a high refractive index; therefore, the episulfide composition of the disclosure can be preferably used for the

production of, for example, an optical material.

The optical material will be described below.

[Polymerizable Composition]

The polymerizable composition of the disclosure contains the above-described episulfide composition of the disclosure and a polymerization catalyst.

The polymerizable composition of the disclosure contains the above-described episulfide composition of the disclosure and is thus excellent in polymerizability.

<Polymerization Catalyst>

The polymerizable composition of the disclosure contains a polymerization catalyst.

The polymerizable composition may contain only a single kind of polymerization catalyst, or may contain two or more kinds of polymerization catalysts.

Examples of the polymerization catalyst include tertiary amine compounds, phosphine compounds, Lewis acids, radical polymerization catalysts, and cationic polymerization catalysts.

With regard to the polymerization catalyst, reference can be made to, for example, the paragraph [0029] to [0033] of JP-A No. 2002-194083.

The polymerization catalyst is preferably a tertiary amine compound or a phosphine compound.

Examples of the tertiary amine compound include triethylamine, tri-*n*-butylamine, tri-*n*-hexylamine, *N,N*-diisopropylethylamine, triethylenediamine, triphenylamine, *N,N*-dimethylethanolamine, *N,N*-diethylethanolamine, *N,N*-dibutylethanolamine, triethanolamine, *N*-ethyldiethanolamine, *N*,*N*-dimethylbenzylamine, *N*,*N*-diethylbenzylamine, tribenzylamine, *N*-methyldibenzylamine, *N*,*N*-dimethylcyclohexylamine, *N*,*N*-dicyclohexylmethylamine, *N,N*-diethylcyclohexylamine, *N*,*N*-dicyclohexylethylamine, *N*,*N*-dimethylbutylamine, *N*,*N*-dicyclohexylbutylamine, *N*-methylmorpholine, *N*-isopropylmorpholine, pyridine, quinoline, *N*,*N*-dimethylaniline, *N*,*N*-diethylaniline, $\alpha$-picoline, $\beta$-picoline, $\gamma$-picoline, 2,2'-bipyridyl, 1,4-dimethylpiperazine, tetramethylethylenediamine, hexamethylenetetramine, 1,8-diazabicyclo(5,4,0)-7-undecene, and 2,4,6-tris(*N*,*N*-dimethylaminomethyl)phenol.

Examples of the phosphine compound include trimethyl phosphine, triethyl phosphine, tri-*n*-propyl phosphine, triisopropyl phosphine, tri-*n*-butyl phosphine, triphenyl phosphine, tribenzyl phosphine, 1,2-bis(diphenylphosphino)ethane, and 1,2-bis(dimethylphosphino)ethane.

In the polymerizable composition of the disclosure, the content of the polymerization catalyst is preferably from 0.01% by mass to 5% by mass, more preferably from 0.01% by mass to 2% by mass, still more preferably from 0.03% by mass to 1% by mass, with respect to a total amount of the polymerizable composition.

<Thiol Compound>

The polymerizable composition of the disclosure preferably further contains a thiol compound.

When the polymerizable composition of the disclosure contains a thiol compound, a resin (i.e., a cured product) can be obtained by polymerizing the episulfide compounds (A) and (B) contained in the episulfide composition with the thiol compound. The resulting resin may have superior resin properties.

When the polymerizable composition of the disclosure contains a thiol compound, the polymerizable composition may contain the thiol compound singly, or in combination of two or more kinds thereof.

The thiol compound may be, for example, an aliphatic thiol or an aromatic thiol.

The aliphatic thiol is preferably at least one selected from the group consisting of pentaerythritol tetrakis(2-mercaptothioglycolate), pentaerythritol tetrakis(3-mercaptopropionate), trimethylolpropane tris(2-mercaptothioglycolate), trimethylolpropane tris(3-mercaptopropionate), 4-mercaptomethyl-1,8-dimercapto-3,6-dithiaoctane, 2,5-dimercaptomethyl-1,4-dithiane, 2,5-bis[(2-mercaptoethyl)thiomethyl]-1,4-dithiane, 1,3-cyclohexane dithiol, 1,4-cyclohexane dithiol, bis(mercaptomethyl)sulfide, bis(mercaptomethyl)disulfide, bis(mercaptoethyl)sulfide, bis(mercaptoethyl)disulfide, 4,8-dimercaptomethyl-1,11-dimercapto-3,6,9-trithiaundecane, 4,7-dimercaptomethyl-1,11-dimercapto-3,6,9-trithiaundecane, 5,7-dimercaptomethyl-1,11-dimercapto-3,6,9-trithiaundecane, and tetrakis(mercaptomethylthio)propane.

However, the aliphatic thiol is not limited to these specific examples.

The aromatic thiol is preferably at least one selected from the group consisting of benzyl thiol, xylylene dithiol, 1,3-bis(mercaptoethyl)benzene, 1,4-bis(mercaptoethyl)benzene, 2,5-toluene dithiol, 3,4-toluene dithiol, thiophenol, 1,2-dimercaptobenzene, 1,3-dimercaptobenzene, 1,4-dimercaptobenzene, and 1,2-bis(mercaptomethyl)benzene.

However, the aromatic thiol is not limited to these specific examples.

The thiol compound is preferably a compound containing two or more thiol groups (i.e., mercapto groups) (hereinafter, also referred to as "polythiol compound").

Further, when the polymerizable composition of the disclosure contains a thiol compound, the thiol compound preferably contains an aliphatic polythiol (i.e., an aliphatic thiol that is a polythiol compound).
In this case, a ratio of the aliphatic polythiol in a total amount of the thiol compound is preferably 50% by mass, more preferably 60% by mass or more, still more preferably 80% by mass or more.
An upper limit of the ratio of the aliphatic thiol is not particularly limited. The ratio of the aliphatic thiol may be 100% by mass, less than 100% by mass, 99% by mass or less, or 95% by mass or less.
The thiol compound more preferably contains at least one selected from the group consisting of 4-mercaptomethyl-1,8-dimercapto-3,6-dithiaoctane, 4,8-dimercaptomethyl-1,11-dimercapto-3,6,9-trithiaundecane, 4,7-dimercaptomethyl-1,11-dimercapto-3,6,9-trithiaundecane, and 5,7-dimercaptomethyl-1,11-dimercapto-3,6,9-trithiaundecane (hereinafter, also referred to as "polythiol A").
In this case, a ratio of the polythiol A in a total amount of the thiol compound is preferably 50% by mass, more preferably 60% by mass or more, still more preferably 80% by mass or more.
An upper limit of the ratio of the polythiol A is not particularly limited. The ratio of the polythiol A may be 100% by mass, less than 100% by mass, 99% by mass or less, or 95% by mass or less.
The thiol compound still more preferably contains at least one selected from the group consisting of 4,8-dimercaptomethyl-1,11-dimercapto-3,6,9-trithiaundecane, 4,7-dimercaptomethyl-1,11-dimercapto-3,6,9-trithiaundecane, and 5,7-dimercaptomethyl-1,11-dimercapto-3,6,9-trithiaundecane (hereinafter, also referred to as "polythiol A1").
In this case, a ratio of the polythiol A1 in a total amount of the thiol compound is preferably 50% by mass, more preferably 60% by mass or more, still more preferably 80% by mass or more.
An upper limit of the ratio of the polythiol A1 is not particularly limited. The ratio of the polythiol A1 may be 100% by mass, less than 100% by mass, 99% by mass or less, or 95% by mass or less.
When the polymerizable composition of the disclosure contains a thiol compound, the content of the thiol compound is preferably from 1% by mass to 50% by mass, more preferably from 2% by mass to 30% by mass, still more preferably from 3% by mass to 20% by mass, yet still more preferably from 5% by mass to 15% by mass, with respect to a total amount of a specific episulfide compound.
With regard to the thiol compound, reference can be made to the paragraph [0039] of WO 2018/079829.

<Other Components>

The polymerizable composition of the disclosure may contain other components in addition to the above-described components.
Examples of the other components include isocyanate compounds, epoxy compounds, alcohol compounds (e.g., polyol compounds), mercapto organic acids, resins (e.g., acrylic resins and olefin resins), crosslinking agents, light stabilizers, UV absorbers, antioxidants, anti-coloring agents, dyes, fillers, and internal mold release agents.
As such other components, any known components can be used.
With regard to the other components, reference can be made as appropriate to, for example, JP-A No. 2002-194083.

<Use>

The use of the polymerizable composition of the disclosure is not particularly limited.
The polymerizable composition of the disclosure can yield a cured product that can exhibit a high refractive index; therefore, the polymerizable composition of the disclosure can be preferably used for the production of, for example, an optical material. The optical material will be described below.

[Cured Product]

The cured product of the disclosure is a cured product of the polymerizable composition of the disclosure.
The cured product of the disclosure can be obtained by, for example, curing the polymerizable composition of the disclosure through polymerization of the monomers contained in the polymerizable composition of the disclosure (e.g., the episulfide compound (A) and the episulfide compound (B), or the episulfide compound (A), the episulfide compound (B), and a thiol compound). In this case, the cured product of the disclosure contains a resin obtained by the polymerization of the monomers contained in the polymerizable composition of the disclosure.
The resulting resin has excellent resin properties.
A method of polymerizing the monomers contained in the polymerizable composition of the disclosure (i.e., a method of producing the cured product of the disclosure) is not particularly limited.
Examples of the method of polymerizing the monomers contained in the polymerizable composition of the disclosure include cast polymerization.

In the cast polymerization, first, the polymerizable composition of the disclosure is injected between casting molds held by a gasket, a tape, or the like. In this process, if necessary, a defoaming treatment, a filtration treatment, and the like may be performed.

Next, the monomers contained in the polymerizable composition injected between the casting molds are polymerized to cure the polymerizable composition between the casting molds and thereby obtain a cured product. Subsequently, the cured product is removed from the casting molds to recover the cured product.

The polymerization of the monomers may also be performed by heating the polymerizable composition of the disclosure. This heating can be performed using, for example, a heating apparatus equipped with a mechanism for heating a material to be heated in an oven, water, or the like.

The conditions for the polymerization of the monomers contained in the polymerizable composition of the disclosure (e.g., polymerization temperature and polymerization time) are set as appropriate, taking into consideration the formulation of the composition, the types and the amounts of the monomers used in the composition, the type and the amount of a polymerization catalyst used in the composition, the shape of the molds, and the like.

The polymerization temperature is, for example, from -50°C to 150°C, or from 10°C to 150°C.

The polymerization time is, for example, from 1 hour to 200 hours, or from 1 hour to 80 hours.

The cured product of the disclosure may also be obtained by performing a treatment such as annealing after the polymerization.

The temperature of this annealing is, for example, from 50°C to 150°C, from 90°C to 140°C, or from 100°C to 130°C.

The cured product of the disclosure is a cured product of a polymerizable composition containing episulfide compounds and, therefore, can have a high refractive index.

The refractive index (nd) of the cured product of the disclosure is preferably 1.71 or higher, more preferably 1.72 or higher, still more preferably 1.73 or higher.

Further, cured products of various shapes can be obtained by changing the casting molds used in the cast polymerization.

Therefore, the cured product of the disclosure can be suitably used as, for example, materials of optical materials, such as eyeglass lenses, camera lenses, and light-emitting diodes (LEDs); and materials of transparent resin members.

[Optical Material]

The optical material of the disclosure includes the cured product of the disclosure.

The optical material of the disclosure may be formed of the cured product of the disclosure, or may include the cured product of the disclosure and other elements.

Examples of the other elements include other members and a coating layer provided on the cured product of the disclosure.

The optical material of the disclosure contains a cured product of an episulfide composition and, therefore, can have a high refractive index.

The refractive index (nd) of the optical material of the disclosure is preferably 1.71 or higher, more preferably 1.72 or higher, still more preferably 1.73 or higher.

Examples of the optical material of the disclosure include eyeglass lenses, camera lenses, polarizing lenses, and light-emitting diodes (LEDs).

-Eyeglass Lens-

An eyeglass lens will now be described as one example of the optical material of the disclosure.

The eyeglass lens includes the cured product of the disclosure that is molded into a desired lens shape.

The eyeglass lens preferably further includes a coating layer provided on one or both sides of the cured product.

Specific examples of the coating layer include a primer layer, a hard coat layer, an anti-reflection layer, an anti-fog coating layer, an anti-fouling layer, and a water-repellent layer. These coating layers may each be used singly, or plural coating layers may be used in the form of a multilayer structure.

When coating layers are provided on both sides of the cured product, the same coating layer may be provided on the respective sides, or different coating layers may be provided on the respective sides.

Components of the coating layer can be selected as appropriate in accordance with the intended purpose.

Examples of the components of the coating layer include a resin (e.g., a urethane resin, an epoxy resin, a polyester resin, a melamine resin, or a polyvinyl acetal resin), an infrared absorber, a light stabilizer, an antioxidant, a photochromic compound, a dye, a pigment, and an antistatic agent.

With regard to the eyeglass lens and the coating layer, reference can be made as appropriate to the descriptions of known documents, such as JP-A No. 2002-194083 and WO 2017/047745.

EXAMPLES

Examples of the disclosure will now be described; however, the disclosure is not limited to the below-described Examples.
In the following description, unless otherwise specified, "part(s)" and "%" are based on mass, "ppm" means ppm by mass, and "wt%" means % by mass.
As methods of analyzing the purity in Examples, the following analysis methods A to C were employed.

[Analysis Method A]

- HPLC apparatus: SPD-10A, manufactured by Shimadzu Corporation
- Measurement wavelength: 210 nm
- Column: YMC A-312 S-5 ODS, 6 mmID × 150 mm
- Temperature condition: 40°C
- Mobile phase: acetonitrile/water = 4/6 (vol/vol)
- Flow rate: 1.0 mL/min
- Sample preparation: 0.2 mL of a reaction solution is collected and dissolved in 10 mL of acetonitrile.
- Injection amount: 2 μL

[Analysis Method B]

The purity was measured using the following measurement conditions.

- HPLC apparatus: SPD-10A, manufactured by Shimadzu Corporation
- Measurement wavelength: 258 nm
- Column: YMC A-312 S-5 ODS, 6 mmID × 150 mm
- Temperature condition: 40°C
- Mobile phase: acetonitrile/water = 4/6 (vol/vol)
- Flow rate: 1.0 mL/min
- Sample preparation: 100 mg of a crude material after desolvation and 100 mg of an internal standard substance are collected and dissolved in 10 mL of acetonitrile.
- Injection amount: 1 μL

[Analysis Method C]

The purity was measured using the following measurement conditions.

- HPLC apparatus: SPD-10A, manufactured by Shimadzu Corporation
- Measurement wavelength: 258 nm
- Column: YMC A-312 S-5 ODS, 6 mmID × 150 mm
- Temperature condition: 40°C
- Mobile phase: acetonitrile/water = 4/6 (vol/vol)
- Flow rate: 1.0 mL/min
- Sample preparation: 150 mg of a crude material after desolvation and 100 mg of an internal standard substance are collected and dissolved in 10 mL of acetonitrile.
- Injection amount: 5 μL

[Synthesis Example 1]

<Synthesis of Episulfide Composition>

To 500 g of methanol, 0.25 g (0.002 mol) of a 48% aqueous NaSH solution and 40.08 g (1.25 mol) of sulfur were added and, while stirring the resultant in a condition where nitrogen was circulated in a gas-phase portion inside a reaction vessel with the internal temperature being maintained at from 5 to 10°C, 161.75 g (1.20 mol) of 94% 1-chloro-3-mercapto-2-propanol was added thereto dropwise over a period of 4 hours. In this process, a 48% aqueous NaSH solution was added dropwise as appropriate such that the pH of the resulting reaction solution was maintained at from

8 to 9.

After a lapse of 4 hours from the completion of the dropwise addition of 161.75 g (1.20 mol) of 94% 1-chloro-3-mercapto-2-propanol, 250 g of methanol and 250 g of pure water were added to the reaction solution, and this reaction solution was aged with stirring for 16 hours while maintaining the internal temperature at from 5 to 10°C. During this aging period as well, a 48% aqueous NaSH solution was added dropwise as appropriate such that the pH of the reaction solution was maintained at from 8 to 9.

After the completion of the aging, the reaction solution was analyzed in accordance with the analysis method A, and it was found that the reaction solution contained 9.2area% of 1,8-dichloro-2,7-dihydroxy-4,5-dithiaoctane and 39.6area% of 1,9-dichloro-2,8-dihydroxy-4,5,6-trithianonane.

It is noted here that each value in area% represents a ratio of the peak area of each component with respect to a total peak area, taking the total peak area as 100area%.

To the reaction solution after the completion of the aging, 500 g of toluene was added in a nitrogen atmosphere, and 249.6 g (1.56 mol) of a 25% aqueous NaOH solution was further added dropwise over a period of 2 hours with the internal temperature being maintained at from 10 to 15°C. The resulting reaction solution was aged for 3 hours with the internal temperature being maintained at from 10 to 15°C, after which 250 g of pure water was added thereto and stirred for 10 minutes to complete the reaction.

After the completion of the reaction, the reaction solution was separated into an organic layer and an aqueous layer, and this aqueous layer was subsequently discharged. While stirring the remaining organic layer, 0.6 g of acetic acid and 25 g of 13% saline water were added thereto to wash the organic layer. The resulting aqueous layer was then discharged and, while stirring the remaining organic layer, 25 g of 13% saline water was added thereto to wash the organic layer. Thereafter, the resulting aqueous layer was discharged, and the remaining organic layer was concentrated, whereby 118.0 g of a crude material containing bis(2,3-epoxypropyl)trisulfide (hereinafter, referred to as "crude material B1") was obtained.

As a result of analyzing the thus obtained crude material B1 by the analysis method B, it was found that the crude material B1 contained 25.5wt% of bis(2,3-epoxypropyl)disulfide and 45.2wt% of bis(2,3-epoxypropyl)trisulfide.

In another flask, 43.3 g (0.83 mol) of 88% formic acid and 63.0 g (0.83 mol) of thiourea were added to 150.0 g of pure water and, with the internal temperature being maintained at 15°C, the above-obtained crude material B1 (118.0 g) was added dropwise to the resultant over a period of 1.5 hours.

After the completion of this dropwise addition, the resultant was further stirred for 3 hours with the internal temperature being maintained at 15°C to obtain a thiuronium chlorination reaction solution.

Subsequently, 160.0 g of methyl isobutyl ketone and 97.0 g of methanol were charged into yet another flask, and the internal temperature thereof was maintained at 10°C.

While maintaining the pH of the resulting mixture at from 7 and 8 and the internal temperature at 10°C, the above-obtained thiuronium chlorination reaction solution and 64.8 g (0.95 mol) of 25% aqueous ammonia were added dropwise to the mixture over a period of 1.5 hours.

The thus obtained reaction solution was stirred for another 2 hours at an internal temperature of 10°C. After the completion of this stirring, the reaction solution was filtered to remove solids, and the resulting filtrate was separated into an organic layer and an aqueous layer, after which the aqueous layer was discharged. The remaining organic layer was washed with an addition of 225.0 g of a 13% aqueous NaCl solution and 2.5 g of acetic acid, and the resulting aqueous layer was subsequently discharged. The remaining organic layer was further washed with an addition of 225.0 g of a 13% aqueous NaCl solution, followed by discharge of the resulting aqueous layer.

The remaining organic layer was vacuum-concentrated, whereby 33.3 g of a crude material containing bis(2,3-epithiopropyl)trisulfide (hereinafter, referred to as "crude material C1") was obtained.

As a result of analyzing the thus obtained crude material C1 by the analysis method C, it was found that the crude material C1 contained 32.9wt% of bis(2,3-epithiopropyl)disulfide as the episulfide compound (B) and 4.5wt% of bis(2,3-epithiopropyl)trisulfide as the episulfide compound (A).

To the crude material C1 (33.3 g), 665.0 g of methylcyclohexane was added, and the resultant was stirred for 1 hour with the internal temperature being maintained at 25°C, after which insoluble components were removed to obtain a methylcyclohexane solution.

A column tube having an inner diameter of 28 mm, into which the whole amount of 16.6 g of silica gel wetted with methylcyclohexane had been charged, was prepared.

Using the thus prepared column tube, the above-obtained methylcyclohexane solution was fractionated by silica gel chromatography, and the resulting fractions were vacuum-concentrated, whereby 12.6 g of an episulfide composition of Synthesis Example 1 was obtained.

As a result of analyzing the thus obtained episulfide composition of Synthesis Example 1 by the analysis method C, it was found that this composition contained 76.4wt% of bis(2,3-epithiopropyl)disulfide as the episulfide compound (B) and 10.6wt% of bis(2,3-epithiopropyl)trisulfide as the episulfide compound (A).

[Synthesis Example 2]

<Synthesis of Episulfide Composition>

To 587.7 g (4.32 mol) of 93% 1-chloro-3-mercapto-2-propanol, 465.5 g of pure water, 399.0 g of sodium bicarbonate, and 131.3 g of methanol were added and, while stirring the resultant in a nitrogen atmosphere with the internal temperature being maintained at 10°C, 372.6 g (2.33 mol) of bromine was added thereto dropwise over a period of 10 hours.

The resulting reaction solution was aged by continuous stirring for 1 hour with the internal temperature being maintained at 10°C.

Next, 450.0 g of toluene was added to the thus aged reaction solution and, while stirring this reaction solution with the internal temperature being maintained at 10°C, 898.1 g (5.61 mol) of a 25% aqueous NaOH solution was added thereto dropwise over a period of 3 hours, after which stirring was continued for another 3 hours with the internal temperature being maintained at 10°C to further age the reaction solution.

Subsequently, 215.9 g of pure water was added thereto, and this reaction solution was stirred for 15 minutes and thereby separated into an organic layer and an aqueous layer, followed by discharge of this aqueous layer. To the remaining organic layer, 30 g of a 13% aqueous NaCl solution was added, and acetic acid was further added with stirring until the resulting aqueous layer exhibited a pH of from 5 to 6. Thereafter, the aqueous layer was discharged.

The remaining organic layer was concentrated under reduced pressure, whereby 372.2 g of a crude material containing bis(2,3-epoxypropyl)disulfide (hereinafter, referred to as "crude material B2") was obtained.

As a result of analyzing the thus obtained crude material B2 by the analysis method B, it was found that the crude material B2 contained 95.1wt% of bis(2,3-epoxypropyl)disulfide; however, bis(2,3-epoxypropyl)trisulfide was not detected.

In another flask, 217.7 g (4.16 mol) of formic acid and 319.2 g (4.16 mol) of thiourea were added to 692.2 g of pure water and, with the internal temperature being maintained at 15°C, the above-obtained crude material B2 (372.2 g, 1.99 mol) was added dropwise to the resultant over a period of 2.9 hours.

After the completion of this dropwise addition, the resultant was further stirred for 3 hours with the internal temperature being maintained at 15°C to obtain a thiuronium chlorination reaction solution.

Subsequently, 752.2 g of methyl isobutyl ketone, 297.6 g (4.37 mol) of 25% aqueous ammonia, and 446.3 g of methanol were charged into yet another flask and, with the internal temperature being maintained at 10°C, the above-obtained thiuronium chlorination reaction solution was added thereto dropwise. Even after the completion of this dropwise addition, stirring was continued for another 2 hours with the internal temperature being maintained at 10°C.

Thereafter, the resulting aqueous layer was discharged, and the remaining organic layer was washed with an addition of 1,056.6 g of a 13% aqueous NaCl solution and 12.0 g of 10% aqueous ammonia, followed by discharge of the resulting aqueous layer. The remaining organic layer was further washed with an addition of 1,056.6 g of a 13% aqueous NaCl solution, 11.8 g of acetic acid, and 343.0 g of methanol, and the resulting aqueous layer was subsequently discharged.

The remaining organic layer was vacuum-concentrated, whereby 436.6 g a crude material containing bis(2,3-epithiopropyl)disulfide (hereinafter, referred to as "crude material C2") was obtained.

As a result of analyzing the thus obtained crude material C2 by the analysis method C, it was found that the crude material C2 contained 81.3wt% (1.69 mol) of bis(2,3-epithiopropyl)disulfide; however, bis(2,3-epithiopropyl)trisulfide was not detected.

Next, 4147.8 g of methylcyclohexane was added to the crude material C2 (436.6 g), and the resultant was stirred for 1 hour with the internal temperature being maintained at 25°C, after which insoluble components were removed. Then, the resultant was stirred for 2 hours at an internal temperature of 15°C, and insoluble components were subsequently removed to obtain a methylcyclohexane solution.

A column tube having an inner diameter of 28 mm, into which the whole amount of 78.6 g of silica gel wetted with methylcyclohexane had been charged, was prepared.

Using the thus prepared column tube, the above-obtained methylcyclohexane solution was fractionated by silica gel chromatography, and the resulting fractions were vacuum-concentrated, whereby 304.4 g of an episulfide composition of Synthesis Example 2 was obtained.

As a result of analyzing the thus obtained episulfide composition of Synthesis Example 2 by the analysis method C, it was found that this composition contained 97.7wt% of bis(2,3-epithiopropyl)disulfide as the episulfide compound (B); however, bis(2,3-epithiopropyl)trisulfide as the episulfide compound (A) was not detected.

<Examples 1 to 4>

Episulfide compositions of Examples 1 to 4 were each obtained by mixing the episulfide composition of Synthesis

Example 1 and the episulfide composition of Synthesis Example 2 such that the content of bis(2,3-epithiopropyl)trisulfide as the episulfide compound (A) and the content of bis(2,3-epithiopropyl)disulfide as the episulfide compound (B) were as shown in Table 1.

<Comparative Example 1>

The episulfide composition of Synthesis Example 2 was used as an episulfide composition of Comparative Example 1.

<Production of Polymerizable Composition>

To 60 g of the episulfide composition of each of Examples 1 to 4 and Comparative Example 1, the following were added:

*N*,*N*-dimethylcyclohexylamine (0.012 g) [polymerization catalyst];
*N*,*N*-dicyclohexylmethylamine (0.060 g) [polymerization catalyst]; and
a mixture S1 (6.0 g) [polythiol composition] containing 4,8-dimercaptomethyl-1,11-dimercapto-3,6,9-trithiaundecane, 4,7-dimercaptomethyl-1,11-dimercapto-3,6,9-trithiaundecane, and 5,7-dimercaptomethyl-1,11-dimercapto-3,6,9-trithiaundecane as main components.

The resultant was stirred at 20°C for 15 minutes to obtain a polymerizable composition of each of Examples 1 to 4 and Comparative Example 1.

<Evaluation of Polymerizability>

The thus obtained polymerizable composition (10 g) of each of Examples 1 to 4 and Comparative Example 1 was placed in a glass container, and a thermometer equipped with a recorder was inserted into the polymerizable composition in the container. In this state, the container was put into an oven and incubated at an oven internal temperature of 30°C for 15 hours to polymerize the monomers (i.e., episulfide compounds and polythiol compounds) contained in the polymerizable composition.

During this polymerization (i.e., 15 hours), the internal temperature of the polymerizable composition was recorded.

Based on the results of recording the internal temperature, the time (h) from the start of the polymerization until the internal temperature reached a maximum temperature was determined. It is noted here that, since the internal temperature of the polymerizable composition rose after the start of the polymerization and eventually fell, a peak top of the internal temperature during the polymerization time (i.e., a temperature at which the internal temperature started to fall) was defined as the maximum temperature.

Based on the thus determined time (h) from the start of the polymerization until the internal temperature reached the maximum temperature (hereafter, this time is referred to as "polymerization rate"), the polymerizability was evaluated in accordance with the following evaluation criteria.

The results thereof are shown in Table 1.

In the following evaluation criteria, "A" is the rank of the most excellent polymerizability.

-Evaluation Criteria of Polymerizability-

A: The polymerization rate is less than 8.5 hours.
B: The polymerization rate is 8.5 hours or more but less than 8.8 hours.
C: The polymerization rate is 8.8 hours or more.

[Table 1]

| | | Unit | Comparative Example 1 | Example 1 | Example 2 | Example 3 | Example 4 |
|---|---|---|---|---|---|---|---|
| Episulfide composition | Episulfide compound (A) | ppm | 0 | 300 | 3410 | 8110 | 11990 |
| | Episulfide compound (B) | wt% | 97.7 | 97.6 | 97.3 | 96.8 | 95.8 |
| Evaluation of polymerizable composition | Polymerizability | | C | A | A | A | A |

As shown in Table 1, the episulfide compositions of Examples 1 to 4, which contained an episulfide compound (A) containing an episulfide ring and a polysulfide bond and an episulfide compound (B) containing an episulfide ring and a (di)sulfide bond, and in which the content of the episulfide compound (A) was 100 ppm by mass or more with respect to a total amount of the respective compositions, had superior polymerizability as compared to the episulfide composition of Comparative Example 1 which did not contain the episulfide compound (A).

Production of Flat Lens>

Flat lenses of Examples 1 to 4 were each produced as cured products of the respective polymerizable compositions of Examples 1 to 4.
The details of a method of producing the flat lenses are as follows.
Each polymerizable composition was injected between a pair of glass molds immobilized by a tape. Next, this pair of glass molds between which the polymerizable composition was thus injected was put into an oven, incubated at an oven internal temperature of 30°C for 15 hours, and then slowly heated such that the oven internal temperature reached 80°C over a period of 10 hours. Subsequently, the pair of glass molds was incubated at an oven internal temperature of 80°C for 2 hours. By this process, the monomers (i.e., episulfide compounds and polythiol compounds) contained in the polymerizable composition were polymerized to form a resin formed body (i.e., a cured product of the polymerizable composition) between the pair of glass molds.
After the above-described 2-hour incubation, the inside of the oven was cooled, and the pair of glass molds was subsequently taken out of the oven, after which the resin formed body (i.e., a pre-annealing flat lens) was obtained by removing it from the pair of glass molds. The thus obtained resin formed body was annealed at 120°C for 3 hours, whereby a circular flat lens (i.e., an annealed flat lens) of 9 mm in thickness and 75 mm in diameter was obtained.

<Measurement of Yellow Index Value (Y.I. Value) of Flat Lens>

As an index of resin property, the yellow index value (Y.I. value) of each flat lens of Examples 1 to 4 was measured before and after the annealing using a color difference meter "CR-400" manufactured by Konica Minolta, Inc.
After the measurement, a value (ΔY.I.) was calculated by subtracting the pre-annealing yellow index value (Y.I. value) from the post-annealing yellow index value (Y.I. value), and the thus calculated ΔY.I. was evaluated as a resin property of each flat lens in accordance with the following evaluation criteria.
The results thereof are shown in Table 2.
In the following evaluation criteria, "A" is the rank of the most excellent ΔY.I. as a resin property.

-Evaluation Criteria of ΔYI.-

A: The ΔY.I. is 1.6 or less.
B: The ΔY.I. is more than 1.6 but 1.8 or less.
C: The ΔY.I. is more than 1.8 but 2.5 or less.
D: The ΔY.I. is more than 2.5.

<Light Resistance Test of Flat Lens>

The surface of each annealed flat lens of Examples 1 to 4 was covered with a UV absorption filter "FUJI FILTER Model No. SC42" manufactured by FUJIFILM Corporation, and the flat lens was maintained in a 7°C atmosphere.
In this state, the surface of the flat lens was irradiated with light emitted from an artificial sun lamp through the UV absorption filter at a cumulative illuminance of 450,000 lux.
The value of a* of the flat lens was measured before and after the irradiation with light using a color difference meter "CR-400" manufactured by Konica Minolta, Inc., and Δa* before and after the irradiation with light was calculated using the following equation:

$$\Delta a^* = (a^* \text{ of flat lens after irradiation}) - (a^* \text{ of flat lens before irradiation})$$

Based on the thus obtained Δa*, the light resistance was evaluated as a resin property of the flat lens in accordance with the following evaluation criteria.
The results thereof are shown in Table 2.
In the following evaluation criteria, "A" is the rank of the most excellent light resistance as a resin property.

-Evaluation Criteria of Light Resistance-

A: The Δa* is less than 0.60.
B: The Δa* is 0.60 or more but less than 0.82.
C: The Δa* is 0.82 or more but less than 0.87.
D: The Δa* is 0.87 or more.

<Measurement of Tg of Flat Lens>

The glass transition temperature (Tg) of each annealed flat lens of Examples 1 to 4 was measured.
As a measurement device, a thermomechanical analyzer "TMA-60" manufactured by Shimadzu Corporation was employed.
Based on the thus obtained results, the Tg was evaluated as a resin property of the flat lens in accordance with the following evaluation criteria.
The results thereof are shown in Table 2.
In the following evaluation criteria, "A" is the rank of the most excellent Tg as a resin property.

-Evaluation Criteria of Tg-

A: The Tg is 81.0°C or higher.
B: The Tg is 79.0°C or higher but lower than 81.0°C.
C: The Tg is 78.0°C or higher but lower than 79.0°C.
D: The Tg is lower than 78.0°C.

[Table 2]

| | | Unit | Example 1 | Example 2 | Example 3 | Example 4 |
|---|---|---|---|---|---|---|
| Episulfide composition | Episulfide compound (A) | ppm | 300 | 3410 | 8110 | 11990 |
| | Episulfide compound (B) | wt% | 97.6 | 97.3 | 96.8 | 95.8 |
| Evaluation of resin properties | Light resistance | | A | A | B | C |
| | ΔY.I. | | A | A | B | C |
| | Tg | | A | A | B | C |

As shown in Table 2, the flat lenses produced using the polymerizable compositions of Examples 1 to 4 had excellent resin properties to some extent (i.e., rank C or better).

The disclosure of Japanese Patent Application No. 2021-142430 filed on September 1, 2021 is hereby incorporated by reference in its entirety.

All the documents, patent applications and technical standards that are described in the present specification are hereby incorporated by reference to the same extent as if each individual document, patent application or technical standard is concretely and individually described to be incorporated by reference.

## Claims

**1.** An episulfide composition, comprising:

an episulfide compound (A) that comprises an episulfide ring, and a polysulfide bond in which three or more bonds represented by -S- are linked together; and

an episulfide compound (B) that comprises an episulfide ring and a (di)sulfide bond but not the polysulfide bond,

wherein a content of the episulfide compound (A) is 100 ppm by mass or more with respect to a total amount of the episulfide composition.

**2.** The episulfide composition according to claim 1, wherein the episulfide compound (A) comprises a trisulfide bond.

**3.** The episulfide composition according to claim 1 or 2, wherein the content of the episulfide compound (A) is 15,000 ppm by mass or less with respect to the total amount of the episulfide composition.

**4.** The episulfide composition according to any one of claims 1 to 3, wherein the content of the episulfide compound (B) is 90% by mass or more with respect to the total amount of the episulfide composition.

**5.** The episulfide composition according to any one of claims 1 to 4, wherein the episulfide compound (A) comprises a compound represented by the following Formula (1):

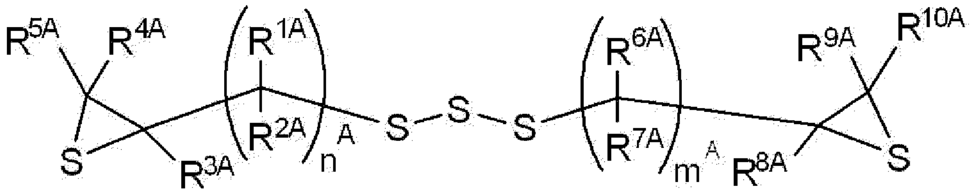

(1)

wherein, in Formula (1), each of $R^{1A}$ to $R^{10A}$ independently represents a hydrogen atom or an optionally substituted monovalent hydrocarbon group, and each of $n^A$ and $m^A$ independently represents an integer from 0 to 3; when $n^A$ is 2 or 3, plural $R^{1A}$s are optionally the same or different, and plural $R^{2A}$s are optionally the same or different; and

when $m^A$ is 2 or 3, plural $R^{6A}$s are optionally the same or different, and plural $R^{7A}$s are optionally the same or different.

**6.** The episulfide composition according to any one of claims 1 to 5, wherein the episulfide compound (B) comprises a compound represented by the following Formula (2):

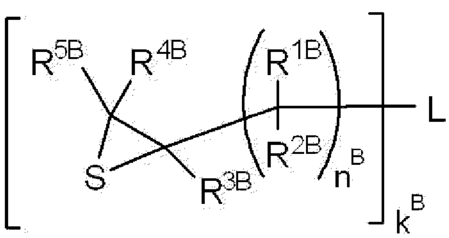

(2)

wherein, in Formula (2), each of $R^{1B}$ to $R^{5B}$ independently represents a hydrogen atom or an optionally substituted

monovalent hydrocarbon group, $n^B$ represents an integer from 0 to 3, and $k^B$ represents an integer from 2 to 4; when $k^B$ is 2, L represents a $k^B$-valent linking group that comprises at least one of a sulfide bond or a disulfide bond and a hydrocarbon group, a sulfide bond, or a disulfide bond;
when $k^B$ is 3 or 4, L represents a $k^B$-valent linking group that comprises at least one of a sulfide bond or a disulfide bond and a hydrocarbon group; and
plural $R^{1B}$s to $R^{5B}$s and $n^B$s are each optionally the same or different.

**7.** A polymerizable composition, comprising:

the episulfide composition according to any one of claims 1 to 6; and
a polymerization catalyst.

**8.** The polymerizable composition according to claim 7, further comprising a thiol compound.

**9.** A cured product of the polymerizable composition according to claim 7 or 8.

**10.** An optical material, comprising the cured product according to claim 9.

## INTERNATIONAL SEARCH REPORT

International application No.
**PCT/JP2022/030174**

**A. CLASSIFICATION OF SUBJECT MATTER**

***C08G 75/08***(2006.01)i; ***C07C 319/24***(2006.01)i; ***C07C 323/24***(2006.01)i; ***C07D 331/02***(2006.01)i; ***G02B 1/04***(2006.01)i
FI: C08G75/08; C07D331/02; C07C323/24; C07C319/24; G02B1/04

According to International Patent Classification (IPC) or to both national classification and IPC

**B. FIELDS SEARCHED**

Minimum documentation searched (classification system followed by classification symbols)

C08G75/08; C07C319/24; C07C323/24; C07D331/02; G02B1/04

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X | JP 6200124 B1 (MITSUI CHEMICALS INC.) 20 September 2017 (2017-09-20) examples, etc. | 1-10 |
| X | JP 2019-104689 A (MITSUI CHEMICALS INC.) 27 June 2019 (2019-06-27) examples, etc. | 1-10 |
| A | JP 2000-281787 A (MITSUBISHI GAS CHEM CO INC) 10 October 2000 (2000-10-10) | 1-10 |
| A | WO 2015/137402 A1 (MITSUI CHEMICALS INC.) 17 September 2015 (2015-09-17) | 1-10 |
| A | JP 11-322930 A (MITSUI CHEMICALS INC.) 26 November 1999 (1999-11-26) | 1-10 |
| A | JP 2002-194083 A (MITSUI CHEMICALS INC.) 10 July 2002 (2002-07-10) | 1-10 |
| A | JP 2000-230050 A (MITSUI CHEMICALS INC.) 22 August 2000 (2000-08-22) | 1-10 |
| A | WO 2015/159811 A1 (MITSUBISHI GAS CHEM CO INC) 22 October 2015 (2015-10-22) | 1-10 |
| A | WO 2010/131631 A1 (MITSUBISHI GAS CHEM CO INC) 18 November 2010 (2010-11-18) | 1-10 |

☐ Further documents are listed in the continuation of Box C.  ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **02 September 2022** | **20 September 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | <br>Telephone No. |

Form PCT/ISA/210 (second sheet) (January 2015)

**INTERNATIONAL SEARCH REPORT**
**Information on patent family members**

International application No.
**PCT/JP2022/030174**

| Patent document cited in search report | Publication date (day/month/year) | Patent family member(s) | Publication date (day/month/year) |
|---|---|---|---|
| JP 6200124 B1 | 20 September 2017 | US 2019/0077751 A1<br>examples, etc.<br>EP 3431466 A1<br>CN 108779068 A<br>KR 10-2018-0113569 A | |
| JP 2019-104689 A | 27 June 2019 | (Family: none) | |
| JP 2000-281787 A | 10 October 2000 | US 6365223 B1<br>EP 1024223 A2 | |
| WO 2015/137402 A1 | 17 September 2015 | US 2017/0015777 A1<br>US 2017/0015776 A1<br>EP 3118195 A1<br>EP 3118233 A1<br>CN 106068260 A<br>KR 10-2016-0125450 A<br>KR 10-2016-0114144 A<br>CN 106232658 A | |
| JP 11-322930 A | 26 November 1999 | US 6204311 B1<br>US 2001/0002413 A1<br>US 2002/0019511 A1<br>EP 942027 A2<br>KR 10-1999-0077861 A | |
| JP 2002-194083 A | 10 July 2002 | US 6204311 B1<br>US 2001/0002413 A1<br>US 2002/0019511 A1<br>EP 942027 A2<br>KR 10-1999-0077861 A | |
| JP 2000-230050 A | 22 August 2000 | US 6204311 B1<br>US 2001/0002413 A1<br>US 2002/0019511 A1<br>EP 942027 A2<br>KR 10-1999-0077861 A | |
| WO 2015/159811 A1 | 22 October 2015 | US 2017/0051095 A1<br>EP 3133098 A1<br>CN 106164120 A<br>KR 10-2016-0147252 A<br>TW 201602195 A | |
| WO 2010/131631 A1 | 18 November 2010 | US 2012/0142889 A1<br>EP 2431401 A1<br>CN 102459418 A<br>KR 10-2012-0031169 A<br>CN 104017216 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2002194083 A
- JP 2021091757 A
- WO 2018079829 A
- WO 2017047745 A
- JP 2021142430 A